(19)

Europäisches
Patentamt
European
Patent Office
Office européen
des brevets

(11) **EP 3 441 783 A1**

(12) **EUROPEAN PATENT APPLICATION**

(43) Date of publication:
**13.02.2019  Bulletin 2019/07**

(21) Application number: **18188586.4**

(22) Date of filing: **10.08.2018**

(51) Int Cl.:
**G01S 13/536** (2006.01)          **G01S 13/88** (2006.01)
**A61B 5/0205** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **10.08.2017   US 201715675002**

(71) Applicant: **Sham, Wellen
Taipei City (TW)**

(72) Inventor: **Sham, Wellen
Taipei City (TW)**

(74) Representative: **Sadler, Peter Frederick
Reddie & Grose LLP
The White Chapel Building
10 Whitechapel High Street
London E1 8QS (GB)**

(54) **IN VEHICLE NON-CONTACT HEARTBEAT AND BREATH SENSING SYSTEM**

(57)    A non-contact sensing system including a bio-radar sensor (206a, 206b) configured to detect a heart rate and/or a respiration rate of a user of a driving apparatus (102) is provided. In some embodiments, a bio-radar sensor (206a, 206b) may be arranged in a seat (202, 204) of the driving apparatus. A processor (208) may be provided in the driving apparatus (102) to process the signals received from the bio-radar sensor (206a, 206b). The processor (208) can monitor the heart rate, respiration rate, and/or any other physiology signs of the user. The processor (208) can compare the detected heart rate and/or the respiration rate of the user with a preset heart rate threshold of the user. The processor (208) can determine whether the heart rate and/or the respiration rate of the user is abnormal. In some embodiments, the processor (208) can be configured to transmit a status indicating the heart rate and/or respiration rate of the user is detected abnormal to a remote control center.

FIG. 2

EP 3 441 783 A1

## Description

CROSS-REFERENCES TO RELATED APPLICATIONS

The present disclosure relates to the following applications: U.S. Nonprovisional Application No. 15/199,268, filed June 30, 2016; U.S. Nonprovisional Application No. 14/977,627, filed December 21, 2015; and U.S. Nonprovisional Application No. 15/063,435, filed March 7, 2016. The entire contents of each of these applications are incorporated by reference herein for all purposes.

BACKGROUND OF THE INVENTION

The present disclosure relates to monitoring a user of a driving apparatus when the user is located inside the driving apparatus.

Traditionally, 'radar', which is an abbreviation for 'RAdio Detection And Ranging', is a system that was developed during World War II and has been used as auxiliary equipment for Air Traffic Control (ATC) and aircraft precision approach. Radar has been installed in airports by the U.S. Federal Aviation Administration (FAA) since the end of the 1940s, and has become major equipment for ATC nowadays.

Radar has an operating principle in which, when radio energy (a short pulse) is emitted from a directional antenna and collides against a target object, waves are reflected, that is, part of the energy returns, and the direction of the target object can be detected using a device for receiving and detecting a reflected waves. That is, radar is equipment for transmitting a radio wave to a target object, receiving the reflected waves of the energy of the radio waves, and measuring the position (direction and distance) of the target object using the round-trip time and the directional characteristics of an antenna based on the straightness and isochronism of a radio wave. Therefore, in the omni-directional emission/reception of a radio wave from/through a ground antenna, the time required for emission/reception is proportional to distance, so that the position of a target object and the distance to the target object can be detected using the direction to the target object. This has been used for the operating principle of distance measurement equipment.

Crashes due to cardio vascular disease while driving represent one possible tragic outcome for individuals with coronary heart disease, and is a source of potential danger to other road users. Sudden cardiac death as a result of an arrhythmia is the most feared complication amongst drivers with existing CVD conditions. Even a benign arrhythmia, for example supraventricular tachycardia (SVT), may cause syncope, and hence incapacitate a driver.

In most places, driving authorities require a driver with a cardio condition that can lead to unsafe driving to follow physical checkups regularly to ensure the driver is still fit to drive. Standards and regulations have been set in many cases to prohibit a driver with certain cardio conditions from driving either temporarily or permanently. However it is common for people with such medical conditions to hide their condition from authorities in order not to be denied a driver's license. Even with the drivers that are known to have medical conditions that can impair their driving performance, risks still exist. For example, such a driver may simply ignore their ailments and choose to drive, and thus potentially put him/her-self and others on the road at risk. Even for a responsible driver, that takes all the necessarily steps to ensure his/her fitness to drive, it is not uncommon to forget to take these steps occasionally. Data has shown most cardio causes of road accidents occur in drivers who are already known to have pre-existing disease.

Accordingly, there is a need to have a heart monitoring system within a driving apparatus to monitor cardio conditions of an operator of the driving apparatus while the operator is operating the driving apparatus.

BRIEF SUMMARY OF THE INVENTION

Embodiments can provide a non-contact sensing system configured to detect a heart rate and/or breathing rate of a user of a driving apparatus. In some embodiments, the non-contact sensing system may be arranged in a seat of the driving apparatus. The non-contact sensing system can include a transmitter that transmits air-propagated ultrasound for monitoring the heart rate and breathing of the user. In some embodiments, the sensing system can comprise at least two ultrasonic transducers, wherein a first transducer is disposed to generate an ultrasonic beam to travel through the air to the user. The ultrasonic beam can be reflected back to a second transducer of the sensing system by a surface of the user skin. Small displacements of the subject's skin surface are caused by breathing, heart motion, and/or any other biometric motion of the user. Subsequently, the first and second transducers can detect variations in the returning echo to derive displacement, velocity, and/or acceleration of the user's skin surface. These displacements can be detected by the system electronics and utilized to determine a plurality of physiological parameters such as heart rate, respiration rate, relative tidal volume, inspiration/expiration times, relative inspiration/expiration flow rates and/or any other physiological parameters. The driving apparatus may be, for example, a vehicle, a train, a ship, or an airplane.

In some embodiments, the bio-radar sensing system can be arranged within a seat of the driving apparatus. In those embodiments, a processing device may be provided in the driving apparatus to process the signals received from the bio-radar sensing system. The processor can be configured to monitor the heart rate, respiration rate, and/or any other aspects of the user's physiology. For example, the processor can be configured to compare the detected heart rate of the user with a preset heart rate threshold of the user. Based on such comparison, the processing device can be configured to determine whether the heart rate of the user is abnormal. Similarly, the processor can be configured to determine whether the respiration rate of the user is abnormal. In some embodiments, the processing device can be configured to transmit a status indicating the heart rate and/or respiration rate of the user is detected abnormal to a remote control center.

In some embodiments, a human machine interaction (HMI) system can be provided in the driving apparatus for visually monitoring the heart rate and/or respiration rate of the user being sensed by the bio-radar sensing system. For example, a display of continuous hear rate and/or respiration rate of the user can be presented on a dashboard screen of the driving apparatus.

This summary is not intended to identify key or essential features of the claimed subject matter, nor is it intended to be used in isolation to determine the scope of the claimed subject matter. The subject matter should be understood by reference to appropriate portions of the entire specification of this patent, any or all drawings, and each claim.

The foregoing, together with other features and embodiments, will become more apparent upon referring to the following specification, claims, and accompanying drawings.

BRIEF DESCRIPTION OF THE DRAWINGS

FIG. 1 generally illustrates an infrastructure for detecting a heart rate and/or respiration rate of a user of a driving apparatus in accordance with the disclosure.

FIG. 2 illustrates bio-radar sensing devices for detecting the heart rate and/or respiration rate of the user of the driving apparatus shown in FIG. 1.

FIG. 3 illustrates one example of a processor that can be equipped within the driving apparatus as shown in FIG. 2.

FIG. 4 illustrates an example of a display that can be equipped within the driving apparatus shown in FIG. 2 to present a real-time graph regarding one or more physiological signs of the user of the driving apparatus.

FIG. 5 illustrates one exemplary method for detecting physiological signs of a user within a driving apparatus in accordance with the disclosure.

FIG. 6 illustrates a simplified computer system according to the disclosure for implementing various embodiments according to the disclosure.

DETAILED DESCRIPTION OF THE INVENTION

Various traditional technologies have been developed to detect a driver's physiological signs. For example, imaging technologies were developed to capture eye movements of the driver to detect whether the driver is drowsy. Some sensing devices were also developed and typically require contact of the driver. For example, some vehicles are equipped with breathalyzers that require the driver to provide breathing gas before he/she can start the vehicles. These technologies typically require an image capturing system or a sensing device positioned very precisely within the vehicle or else requires contact of the driver for detecting the physiology signs of the driver. In many cases, such requirements render these technologies prone to mistakes. For example, the imaging system may not be able capture images of eyes of the driver if he or she is not seated in a position to be capture-able - for instance, the driver may be too tall or too short. Of course, the contact sensing devices will not work properly, when there is no contact from the driver.

In accordance with the disclosure, embodiments can provide a contact-less sensing system that uses a bio-radar component for detecting a heart rate, respiration rate and/or any other physiological signs of a user (e.g., a driver or a passenger) of a driving apparatus. The sensing system may be arranged within a seat of the driving apparatus and may be configured to acquire displacement changes of the user's heart and/or respiration motion. A processing device may be provided in the driving apparatus to process the signals received from the sensing system. The processor can be configured to monitor the heart rate, respiration rate, and/or any other aspects of the user's physiology. The processing device can be configured to determine whether the heart rate, respiration rate and/or any other physiological sign of the user is abnormal. In some embodiments, the processing device can be configured to transmit a status indicating the heart rate and/or respiration rate of the user is detected abnormal to a remote control center.

In some embodiments, a human machine interaction (HMI) system can be provided in the driving apparatus for visually monitoring the heart rate and/or respiration rate of the user being sensed by the sensing system. For example, a display of continuous hear rate and/or respiration rate of the user can be presented on a dashboard screen of the driving apparatus.

Various specific embodiments of the present disclosure will be described below with reference to the accompanying drawings constituting a part of this specification. It should be understood that, although structural parts and components of various examples of the present disclosure are described by using terms expressing directions, e.g., "front", "back", "upper", "lower", "left", "right" and the like in the present disclosure, these terms are merely used for the purpose of convenient description and are determined on the basis of exemplary directions displayed in the accompanying drawings. Since the embodiments disclosed by the present disclosure may be set according to different directions, these terms expressing directions are merely used for describing rather than limiting. Under possible conditions, identical or similar reference numbers used in the present disclosure indicate identical components.

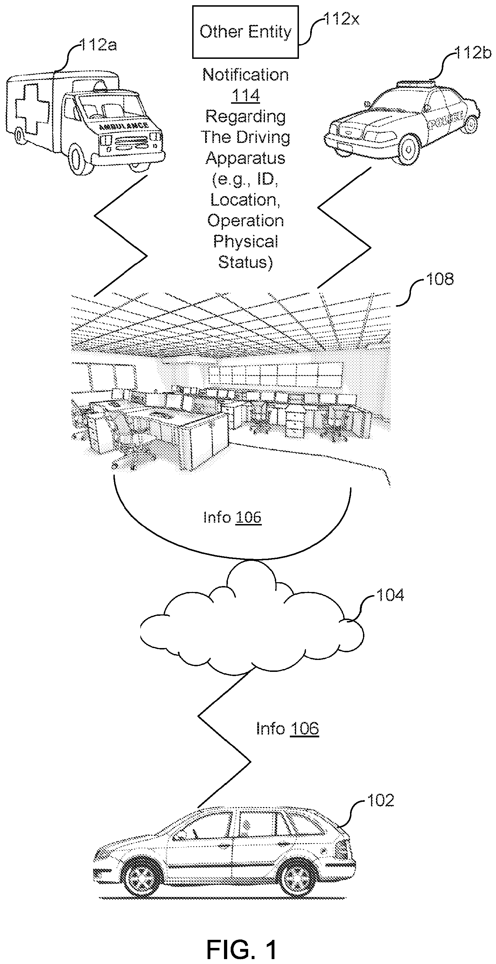

FIG. 1 generally illustrates an infrastructure for detecting a heart rate and/or respiration rate of a user of a driving apparatus 102 in accordance with the disclosure. The driving apparatus 102 may include any driving apparatus that moves in distance. Examples of driving apparatus 102 may include a vehicle such as an ambulance, a car, a bus, a train, a truck, a tram, or any other type of vehicle; may include a vessel such as a boat, a ship, a barge, a ferry or any other type of watercraft; may include an aircraft such as an airplane, a spaceship, or any other type of aircraft; or may include any other transportation apparatus. In one example, the driving apparatus 102 is an electrical automobile. As shown, the driving apparatus 102 can generate information 106 from time to time. The information 106 can include a status indicating an abnormal physiological condition ("abnormal condition status") having been detected for the user of the driving apparatus 102, and/or any other status information regarding the user of the driving apparatus 102. The information 106 can include identification information regarding the user of the driving apparatus 102, and/or the apparatus 102. The abnormal condition status generated by driving apparatus 102 can indicate a heart rate of the operator is below a heart rate threshold, a respiration rate of the user is below a respiration threshold, and/or any other physiological conditions of the operator of the driving apparatus having been detected.

As shown, in some embodiments, the some or all of the information 106 can be transmitted from driving apparatus 102 to a control center 108 via a network 104. The network 104 can employ a wireless transmission technology such as ultra high frequency radio, cellular, WIFI, bluetooth, infrad, laser, and/or any other wirless transmission technology. Information 106 can be transmitted through network 104 to the control center 108. The control center 108 can house one or more servers. The one or more servers in the control center 108 can be configured to receive information 106. The one or more servers in the control center 108 can be configured to generate a notification in response to the abnormal heart rate and/or respiration rate of the driver having been detected. In certain embodiments, the one or more servers in the control center 108 can be configured to determine a risk level in response to the fatigue condition of the driver having been detected, and/or any other information. In certain embodiments, the one or more server in the control center 108 can be configured to generate notifications 114 based on the determined risk level. The notifications can include a notification to a human operator in the control center 108, a notification to a healthcare facility 112a, a law enforcement agency 112b, an emergency contact for the operator of driving apparatus 102, an insurance agency, and/or any other entities. The notifications 114 can include identification information regarding the user.

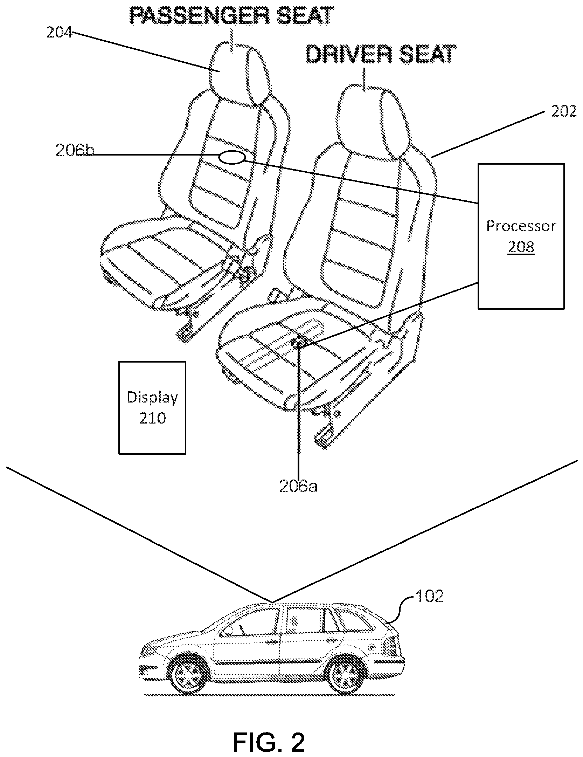

With the infrastructure for detecting a heart rate and/or respiration rate of the user of the driving apparatus 102 having been generally described, attention is now directed to FIG. 2. FIG. 2 illustrates bio-radar sensing devices can be placed within seats of driving apparatus 102 for detecting the heart rate and/or respiration rate of the user of the driving apparatus 102. As shown, the driving apparatus 102 can have a driver seat 202 and/or a passenger seat 204. In this embodiment, both the driver seat 202 and the passenger seat 204 are equipped with a bio-radar sensor 206a and 206b, respectively. Any one of the bio-radars 206a and 206b can comprise a transmitter that transmits air-propagated ultrasound for monitoring the heart rate and breathing of the user. In some embodiments, the transmitter can comprise at least two ultrasonic transducers, wherein a first transducer is disposed to generate an ultrasonic beam to travel through the air to the user, e.g., driver or passenger. The ultrasonic beam can be reflected back to a second transducer of the sensing system by a surface of the user's skin. Small displacements of the user's skin surface caused by breathing, heart motion, and/or any other biometric motion of the user can be detected through fluctuations of the ultrasonic beam over time. The first and/or second transducers can detect variations in the returning echo to derive displacement, velocity, and/or acceleration of the user's skin surface.

In some embodiments, the bio-sensor radar 206a and/or 206b can comprise electronics, e.g., circuits configured to detect these displacements and to determine a plurality of physiological parameters such as heart rate, respiration rate, relative tidal volume, inspiration/expiration times, relative inspiration/expiration flow rates and/or any other physiological parameters of the user. In some embodiments, as shown in this example, the bio-radar 206a and/or 206b can be arranged within a seat 204 of the driving apparatus. In those embodiments, a processing device 208 may be provided in the driving apparatus to process the signals received from the bio-radar 206a and/or 206b. The processor 208 can be configured to monitor the heart rate, respiration rate, and/or any other aspects of the user's physiology. For example, the processor 208 can be configured to compare the detected heart rate of the user with a preset heart rate threshold of the user. Based on such comparison, the processor 208 can be configured to determine whether the heart rate of the user is abnormal. Similarly, the processor can be configured to determine whether the respiration rate of the user is abnormal. In some embodiments, the processing device can be configured to transmit a status indicating the heart rate and/or respiration rate of the user is detected abnormal to a remote control center.

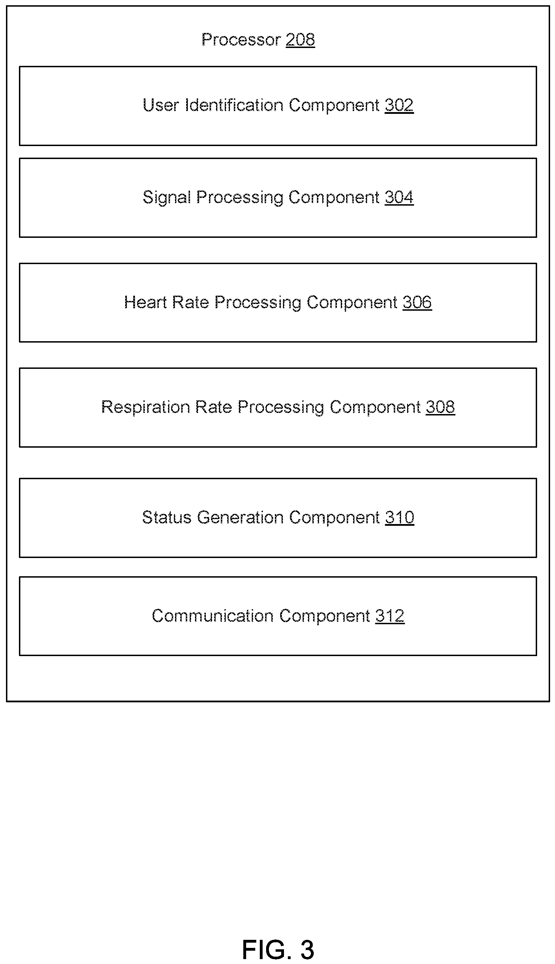

FIG. 3 illustrates one example of a processor 208 that can be equipped within the driving apparatus 102 as shown in FIG. 2. The processor 208 may be configured to execute program components including a user identification component 302, signal processing component 304, heart rate processing component 306, respiration rate processing component 308, status generation processing component 310, communication component 312, and/or any other components. The processor 208 may be wirelessly connected or connected by wire to the bio-radars 206a and/or 206b. The processor 208 may receive and/or obtain signals generated by the bio-radars 206a and/or 206b periodically or non-periodically.

The user identification component 208 can be configured to identify the operator(s) and/or the passenger(s) of the driving apparatus 102. In some embodiments, the identification of the operator(s) and/or passenger(s) of the driving apparatus 102 by the user identification component 302 can be made based on the fingerprint image acquired by a fingerprint detection component provided in the driving apparatus 102, the image of operator's face captured by an image capturing device arranged within the driving apparatus 102, and/or any other identification information regarding the operator(s) and/or the passenger(s). For example, the identification by the user identification component 302 may involve analyzing features in the fingerprint image and/or in the facial image of a driver of the driving apparatus 102, and comparing the obtained features with features of registered operators. Upon a match, the user identification component 302 can be configured to obtain a user ID of the identified operator.

The signal processing component 304 can be configured to process the signals generated by the bio-radar 206a and/or 206b. Typically, as described above, the bio-radars 206a and/or 206b can transmit a continuous-wave (CW) signal to the user and demodulate the CW signal reflected off of the user's skin surface, e.g., the surface of the chest of the user. Consistent with Doppler theory, a human chest-wall has a time-varying position with net zero velocity and will reflect a signal with its phase-modulated in proportion to the position of the chest-wall. In some embodiments, the signal processing component 304 can be configured to process the demodularized signals generated by the bio-radars 206a and/or 206b. The signal processing by the signal processing component 304 can include determining a signal-to-noise ratio (SNR) value. In implementations, the SNR value determination by the signal processing component 304 may involve using a simple path loss value and various noise effects at the baseband output as design variables. The SNR value determined by the signal processing component 304 can be particularly important when measuring the motion due to heartbeat, since the information is encoded in phase modulations of 0.1 to 10 Hz, where the phase noise is near the peak. In order to calculate the SNR value, the signal processing component 304 may be configured to determine the magnitude of the signal received from the bio-radars 206a and/or 206b and the noise power. The magnitude of the signal at the baseband depends on the received power and phase modulation. Noise sources include residual phase noises from the LO, additive white Gaussian noise (AWGN), and the baseband 1/f noise of the mixer and of the baseband analog circuits.

In some embodiments, the signals received from the bio-radars 206a and/or 206b may include mixed heartbeat and respiration signals. In those embodiments, the signal processing component 304 can be configured to separate the heartbeat and respiration signals. In some implementations, additional electronics, such as circuits, may be provided with the processor 208 for separating the heartbeat and respiration signals. For example, a baseband module can be provided to separate the heartbeat and respiration signals received from the bio-radars 206a and/or 206b. The baseband module consists of an offset circuit, a band pass filter circuit, and an amplifier to separate the heartbeat and respiration. The band pass filter for respiration has a frequency bandwidth of 0.05 to 0.5 Hz. The band pass filter for heartbeat has a frequency bandwidth of 1 to 30 Hz.

The heart rate processing component 306 can be configured to determine a heart rate of the user based on the heart rate signals provided by the signal processing component 304. The signal displacement generated by the user as detected by the bio-radars 206a and/or 206b can consist of heartbeat of the user, which is typically in the range 0.8-2 Hz. The heart rate signal is a complicated signal and nearly periodic, but the period can vary from one beat to the next. This is called heart rate variability (HRV). The heartrate determination by the heart rate processing component 306 can involve using a random process with strong periodicity. The heart rate signal can be modeled using following formula:

$$s(t) = (A + \alpha(t)) \cos{(\omega t + \theta(t) + \theta_0)},$$

where $\varphi(t)$ is phase noise in the oscillator or bio-radar 206a or 206b, $\omega$ is the single tone signal frequency at which the CW wave is transmitted by the bio-radar 206a or 206b, A is a constant, and $\alpha(t)$ and $\theta(t)$ are amplitude variations having zero mean random for modeling the HRV. In implementations, $\theta(t)$ can be approximated. As $\theta(t)$ is rapidly varying (the time between heartbeats can vary 10% from one heartbeat to the next), a piecewise constant approximation can be used.

The respiration rate processing component 308 can be configured to determine a respiration rate of the user based on the respiration rate signals provided by the signal processing component 304. The respiration of the user is usually in the range 0.1-0.8 Hz and is a stronger signal than the heartbeat. In implementations, the respiration rate processing component 308 can be configured to perform filtering of the respiration signals to increase the SNR of noisy RSS measurements r(n) and down-sampled by a decimation factor M to decrease the computational requirements of the system. The respiration rate processing component 308 can be configured to apply sampled signals y(k) using spectral estimation techniques and to make the system environment independent so that training is not required. The respiration rate processing component 308 can be configured to monitor the mean removed RSS measurements, and the state of the system to enable breathing estimation only when the process is stationary.

The status generation component 310 can generate a status to indicate that an abnormal heart rate and/or respiration rate is detected for the user based on the heart rate and/or the respiration rate values determined by the heart rate processing component 306 and respiration rate processing component 308. In implementations, the status generation component 310 configured to may be configured to retrieve one or more predetermined thresholds for generating the status. For example, the status generation component 310 can be configured to compare the detected heart rate of the user with a preset heart rate threshold of the user. Different users may be associated with different preset heart rate thresholds and personalized heart rate threshold can be retrieved by the status generation processing component 310 based on the user identification made by the user identification component 302. Based on such comparison, the status generation component 310 can be configured to determine whether the heart rate of the user is abnormal. Similarly, the status generation component 310 can be configured to determine whether the respiration rate of the user is abnormal by comparing the detected respiration rate with a preset respiration rate for the user.

The communication component 312 can be configured to communicate the status generated by the status generation component 310, and/or any other information to a control center, such as control center 108, and/or any other entities. The communication component 312 can be configured to communicating such information via a communications network.

It should be understood the above-described functionalities attributed to processor 208 can be implemented within the driving apparatus 102. However, this is not necessarily the only case. In certain embodiment, part of or the entire functionalities attributed to processor 208 herein can be implemented at the control center, for example control center 108. For example, the control center 108 may comprise a server that can be configured to perform part of the operations provided by processor 208 as described above.

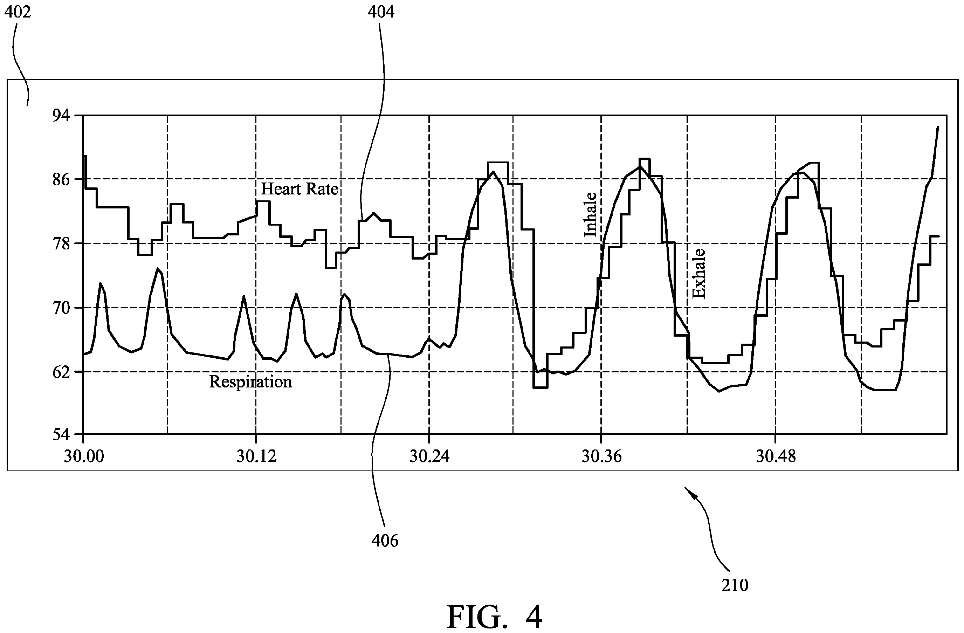

Returning to FIG. 2, in some embodiments, the driving apparatus 102 may be equipped with a display 210 configured to present real-time heart rate and/or respiration rate of the operator(s) and/or passenger(s) of the driving apparatus 102. The display 210 may include a CRT, LCD, LED or OLED screen mounted at a dashboard of the driving apparatus 106. FIG. 4 illustrates an example of the display 210. As shown, the display 210 can present a real-time graph 402 regarding one or more physiology conditions of the user. The real-time graph 402 can include a graph 404 showing a real-time heart rate of the user as detected by the bio-radars 206a and/or 206b and determined by the processor 208. The real-time graph 402 can include a graph 406 showing a real-time respiration rate of the user as detected by the bio-radars 206a and/or 206b and determined by the processor 208.

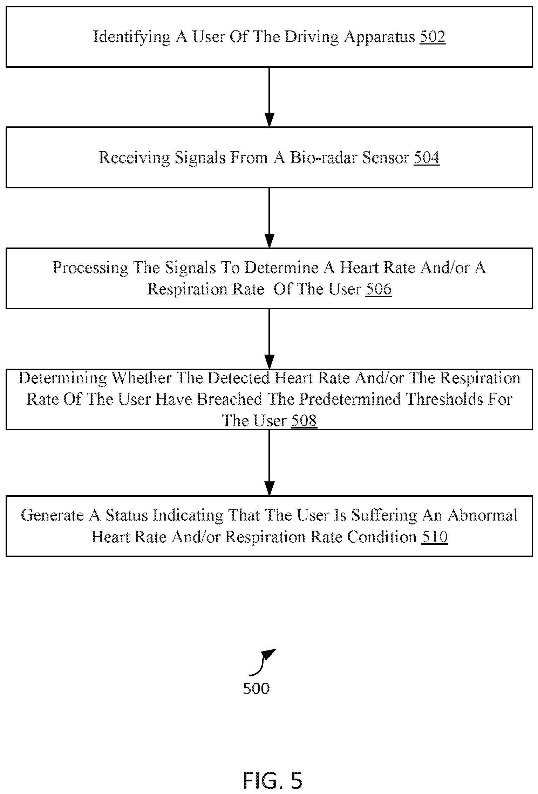

FIG. 5 illustrates one exemplary method for detecting physiological signs of a user within a driving apparatus in accordance with the disclosure. The operations of method 500 presented below are intended to be illustrative. In some embodiments, method 500 may be accomplished with one or more additional operations not described and/or without one or more of the operations discussed. Additionally, the order in which the operations of method 500 are illustrated in FIG. 5 and described below is not intended to be limiting.

In some embodiments, method 500 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 500 in response to instructions stored electronically on an electronic storage medium. The one or more processing devices may include one or more devices configured through hardware, firmware, and/or software to be specifically designed for execution of one or more of the operations of method 500.

At 502, a user of the driving apparatus can be identified. In some implementations, operation 502 can be performed by a user identification component substantially similar to or the same as the user identification component 302 as described and illustrated herein.

At 504, signals can be received from a bio-radar sensor located in a seat of the driving apparatus. An example of the bio-radar sensor is illustrated in FIG. 2. Typically, as described above, the bio-radar sensor can transmit a continuous-wave (CW) signal to the user and demodulate the CW signal reflected off of the user's skin surface, e.g., the surface of the chest of the user. In some embodiments, the signals received from the bio-radar sensor may include signals for the heart rate and/or the respiration of the user. In some implementations, operation 504 can be performed by signal processing component substantially similar to or the same as the signal processing component 304 as described and illustrated herein.

At 506, the signals received at 504 can be processed to determine a heart rate and/or a respiration rate of the user. The signal processing at 504 can include determining a signal-to-noise ratio (SNR) value for the received signals. As mentioned above, in some embodiments, the signals received from the bio-radar sensor may include mixed heartbeat and respiration signals. In those embodiments, the signal processing at 504 can involve separating the heartbeat and respiration signals. In some implementations, additional electronics, such as circuits, may be used for separating the heartbeat and respiration signals. In some implementations, operations involved in 508 can be performed by a signal processing component, a heart rate processing component and/or a respiration rate processing component the same as or substantially similar to signal processing component 304, a heart rate processing component 306 and/or a respiration rate processing component 308 described and illustrated herein.

At 508, a determination whether the detected heart rate and/or the detected respiration rate of the user have breached corresponding thresholds can be determined. In some embodiments, different users may be associated with different preset heart rate and/or respiration rate thresholds and personalized heart rate can be retrieved by the status generation processing component 310 based on the user identification made at 502. In some implementations, operation 508 can be performed by status generation processing component the same as or substantially similar to status generation processing component 310 described and illustrated herein.

At 510, in response to determining that the detected heart rate and/or the detected respiration rate of the user have breached corresponding thresholds, a status can be generated to indicate such. In some implementations, the status generated at 510 can be transmitted to a computing device at a control center via a network (e.g., a mixture of a wired and wireless network). In some implementations, operation 510 can be performed by status generation processing component the same as or substantially similar to status generation processing component 310 described and illustrated herein.

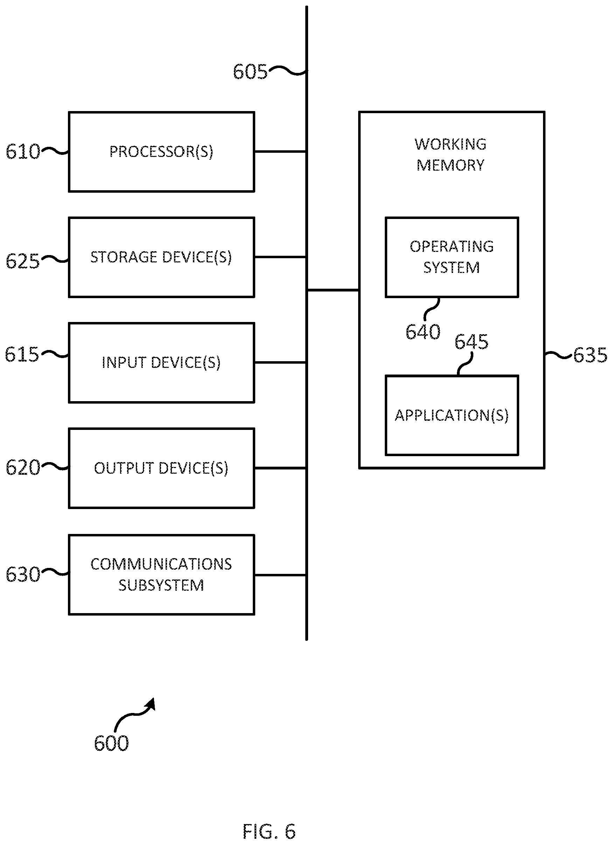

FIG. 6 illustrates a simplified computer system, according to the present disclosure for implementing various embodiments according to the present disclosure. A computer system 600 as illustrated in FIG. 6 may be incorporated into devices such as a portable electronic device, mobile phone, or other device as described herein. FIG. 6 provides a schematic illustration of one embodiment of a computer system 600 that can perform some or all of the steps of the methods provided by various embodiments. It should be noted that FIG. 6 is meant only to provide a generalized illustration of various components, any or all of which may be utilized as appropriate. FIG. 6, therefore, broadly illustrates how individual system elements may be implemented in a relatively separated or relatively more integrated manner.

The computer system 600 is shown comprising hardware elements that can be electrically coupled via a bus 605, or may otherwise be in communication, as appropriate. The hardware elements may include one or more processors 610, including without limitation one or more general-purpose processors and/or one or more special-purpose processors such as digital signal processing chips, graphics acceleration processors, and/or the like; one or more input devices 615, which can include without limitation a mouse, a keyboard, a camera, and/or the like; and one or more output devices 620, which can include without limitation a display device, a printer, and/or the like.

The computer system 600 may further include and/or be in communication with one or more non-transitory storage devices 625, which can comprise, without limitation, local and/or network accessible storage, and/or can include, without limitation, a disk drive, a drive array, an optical storage device, a solid-state storage device, such as a random access memory ("RAM"), and/or a read-only memory ("ROM"), which can be programmable, flash-updateable, and/or the like. Such storage devices may be configured to implement any appropriate data stores, including without limitation, various file systems, database structures, and/or the like.

The computer system 600 might also include a communications subsystem 630, which can include without limitation a modem, a network card (wireless or wired), an infrared communication device, a wireless communication device, and/or a chipset such as a Bluetooth™ device, an 802.11 device, a WiFi device, a WiMax device, cellular communication facilities, etc., and/or the like. The communications subsystem 630 may include one or more input and/or output communication interfaces to permit data to be exchanged with a network such as the network described below to name one example, other computer systems, television, and/or any other devices described herein. Depending on the desired functionality and/or other implementation concerns, a portable electronic device or similar device may communicate image and/or other information via the communications subsystem 630. In other embodiments, a portable electronic device, e.g. the first electronic device, may be incorporated into the computer system 600, e.g., an electronic device as an input device 615. In some embodiments, the computer system 600 will further comprise a working memory 635, which can include a RAM or ROM device, as described above.

The computer system 600 also can include software elements, shown as being currently located within the working memory 635, including an operating system 660, device drivers, executable libraries, and/or other code, such as one or more application programs 665, which may comprise computer programs provided by various embodiments, and/or may be designed to implement methods, and/or configure systems, provided by other embodiments, as described herein. Merely by way of example, one or more procedures described with respect to the methods discussed above, such as those described in relation to FIG. 6, might be implemented as code and/or instructions executable by a computer and/or a processor within a computer; in an aspect, then, such code and/or instructions can be used to configure and/or adapt a general purpose computer or other device to perform one or more operations in accordance with the described methods.

A set of these instructions and/or code may be stored on a non-transitory computer-readable storage medium, such as the storage device(s) 625 described above. In some cases, the storage medium might be incorporated within a computer system, such as computer system 600. In other embodiments, the storage medium might be separate from a computer system e.g., a removable medium, such as a compact disc, and/or provided in an installation package, such that the storage medium can be used to program, configure, and/or adapt a general purpose computer with the instructions/code stored thereon. These instructions might take the form of executable code, which is executable by the computer system 600 and/or might take the form of source and/or installable code, which, upon compilation and/or installation on the computer system 600 e.g., using any of a variety of generally available compilers, installation programs, compression/decompression utilities, etc., then takes the form of executable code.

It will be apparent to those skilled in the art that substantial variations may be made in accordance with specific requirements. For example, customized hardware might also be used, and/or particular elements might be implemented in hardware, software including portable software, such as applets, etc., or both. Further, connection to other computing devices such as network input/output devices may be employed.

As mentioned above, in one aspect, some embodiments may employ a computer system such as the computer system 600 to perform methods in accordance with various embodiments of the technology. According to a set of embodiments, some or all of the procedures of such methods are performed by the computer system 600 in response to processor 610 executing one or more sequences of one or more instructions, which might be incorporated into the operating system 660 and/or other code, such as an application program 665, contained in the working memory 635. Such instructions may be read into the working memory 635 from another computer-readable medium, such as one or more of the storage device(s) 625. Merely by way of example, execution of the sequences of instructions contained in the working memory 635 might cause the processor(s) 610 to perform one or more procedures of the methods described herein. Additionally or alternatively, portions of the methods described herein may be executed through specialized hardware.

The terms "machine-readable medium" and "computer-readable medium," as used herein, refer to any medium that participates in providing data that causes a machine to operate in a specific fashion. In an embodiment implemented using the computer system 600, various computer-readable media might be involved in providing instructions/code to processor(s) 610 for execution and/or might be used to store and/or carry such instructions/code. In many implementations, a computer-readable medium is a physical and/or tangible storage medium. Such a medium may take the form of a non-volatile media or volatile media. Non-volatile media include, for example, optical and/or magnetic disks, such as the storage device(s) 625. Volatile media include, without limitation, dynamic memory, such as the working memory 635.

Common forms of physical and/or tangible computer-readable media include, for example, a floppy disk, a flexible disk, hard disk, magnetic tape, or any other magnetic medium, a CD-ROM, any other optical medium, punchcards, papertape, any other physical medium with patterns of holes, a RAM, a PROM, EPROM, a FLASH-EPROM, any other memory chip or cartridge, or any other medium from which a computer can read instructions and/or code.

Various forms of computer-readable media may be involved in carrying one or more sequences of one or more instructions to the processor(s) 610 for execution. Merely by way of example, the instructions may initially be carried on a magnetic disk and/or optical disc of a remote computer. A remote computer might load the instructions into its dynamic memory and send the instructions as signals over a transmission medium to be received and/or executed by the computer system 600.

The communications subsystem 630 and/or components thereof generally will receive signals, and the bus 605 then might carry the signals and/or the data, instructions, etc. carried by the signals to the working memory 635, from which the processor(s) 610 retrieves and executes the instructions. The instructions received by the working memory 635 may optionally be stored on a non-transitory storage device 625 either before or after execution by the processor(s) 610.

The methods, systems, and devices discussed above are examples. Various configurations may omit, substitute, or add various procedures or components as appropriate. For instance, in alternative configurations, the methods may be performed in an order different from that described, and/or various stages may be added, omitted, and/or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Specific details are given in the description to provide a thorough understanding of exemplary configurations including implementations. However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the spirit or scope of the disclosure.

Also, configurations may be described as a process which is depicted as a schematic flowchart or block diagram. Although each may describe the operations as a sequential process, many of the operations can be performed in parallel or concurrently. In addition, the order of the operations may be rearranged. A process may have additional steps not included in the figure. Furthermore, examples of the methods may be implemented by hardware, software, firmware, middleware, microcode, hardware description languages, or any combination thereof. When implemented in software, firmware, middleware, or microcode, the program code or code segments to perform the necessary tasks may be stored in a non-transitory computer-readable medium such as a storage medium. Processors may perform the described tasks.

Having described several example configurations, various modifications, alternative constructions, and equivalents may be used. For example, the above elements may be components of a larger system, wherein other rules may take precedence over or otherwise modify the application of the technology. Also, a number of steps may be undertaken before, during, or after the above elements are considered.

As used herein and in the appended claims, the singular forms "a", "an", and "the" include plural references unless the context clearly dictates otherwise. Thus, for example, reference to "a user" includes a plurality of such users, and reference to "the processor" includes reference to one or more processors and equivalents thereof known to those skilled in the art, and so forth.

Also, the words "comprise", "comprising", "contains", "containing", "include", "including", and "includes", when used in this specification and in the following claims, are intended to specify the presence of stated features, integers, components, or steps, but they do not preclude the presence or addition of one or more other features, integers, components, steps, acts, or groups.

## Claims

1. A method for automatically detecting physiological signs of a user within a driving apparatus, the method being implemented in one or more processors configured to execute machine-readable instructions, the method comprising:

   identifying a user of the driving apparatus;
   receiving signals from a first bio-radar sensor, the first bio-radar sensor being equipped in a first seat of the driving apparatus and the signals including information regarding a heart rate of the user;
   in response to receiving the signals from the first bio-radar sensor, processing the signals to determine a heart rate value of the user;
   comparing the heart value of the user with a predetermined heart rate threshold for the user;
   determining whether the detected heart rate of the user has breached the predetermined heart rate threshold for the user; and
   in response to determining the detected heart rate of the user has breached the predetermined heart rate threshold for the user, generate a first status indicating that the user is suffering an abnormal heart rate condition.

2. The method of claim 1, wherein the signals received from the first bio-radar sensor further comprise information regarding a respiration rate of the user; and, wherein the method further comprises:

   processing the signals received from the bio-radar sensor to separate a heart rate signal and a respiration signal of the user.

3. The method of claim 2, further comprising:

   determining a respiration rate value of the user;
   comparing the respiration rate value of the user with a predetermined respiration rate threshold for the user; and
   determining whether the detected respiration rate of the user has breached the predetermined respiration rate threshold for the user; and, wherein
   the generated first status further indicates that the user is suffering an abnormal respiration rate condition, or a status is generated indicating that the user is suffering an abnormal respiration rate condition.

4. The method of any of claims 1, 2, or 3, further comprising
   transmitting the first status to a computing device located in a control center over a network.

5. The method of any of the above claims, wherein the processing of the signals received from the first bio-radar sensor includes determining a signal to noise (SNR) value of the signals.

6. The method of any of the above claims, wherein determining the heart rate value of the user includes modeling the heart rate of the user according to a random process.

7. The method of any of the above claims, further comprising presenting the heart rate value on a display located within the driving apparatus.

8. The method of any of the above claims, wherein the user is a driver of the driving apparatus; and wherein the method further comprises:

identifying a passenger of the driving apparatus;
receiving signals from a second bio-radar sensor, the second bio-radar sensor being equipped in a second seat of the driving apparatus and the signals include information regarding a heart rate of the passenger;
in response to receiving the signals from the second bio-radar sensor, processing the signals to determine a heart rate value of the passenger;
comparing the heart value of the passenger with a predetermined heart rate threshold for the passenger;
determining whether the detected heart rate of the passenger has breached the predetermined heart rate threshold for the passenger; and
in response to determining the detected heart rate of the passenger has breached the predetermined heart rate threshold for the passenger, generate a second status indicating that the passenger is suffering an abnormal heart rate condition.

9. A system for automatically detecting physiological signs of a user within a driving apparatus, the system comprising one or more processors configured to execute machine-readable instructions such that when the machine-readable instructions are executed, the one or more processors are caused to perform:

identifying a user of the driving apparatus;
receiving signals from a first bio-radar sensor, the bio-radar sensor being equipped in a first seat of the driving apparatus and the signals include information regarding a heart rate of the user;
in response to receiving the signals from the first bio-radar sensor, processing the signals to determine a heart rate value of the user;
comparing the heart value of the user with a predetermined heart rate threshold for the user;
determining whether the detected heart rate of the user has breached the predetermined heart rate threshold for the user; and
in response to determining the detected heart rate of the user has breached the predetermined heart rate threshold for the user, generate a first status indicating that the user is suffering an abnormal heart rate condition.

10. The system of claim 9, wherein the signals received from the first bio-radar sensor further comprise information regarding a respiration rate of the user; and, wherein the system further comprises:

processing the signals received from the first bio-radar sensor to separate a heart rate signal and a respiration signal of the user.

11. The system of claim 10, wherein the processor is further caused to perform:

determining a respiration rate value of the user;
comparing the respiration rate value of the user with a predetermined respiration rate threshold for the user; and
determining whether the detected respiration rate of the user has breached the predetermined respiration rate threshold for the user; and, wherein
the generated first status indicates that the user is suffering an abnormal respiration rate condition.

12. The system of any of claims 9, 10, or 11, wherein the processor is further caused to perform:

transmitting the status to a computing device located in a control center over a network.

13. The system of any of claims 9 to 12, wherein the processing of the signals received from the first bio-radar sensor includes determining a signal to noise (SNR) value of the signals, and/or wherein determining the heart rate value of the user includes modeling the heart rate of the user according to a random process.

14. The system of any of claims 9 to 13, wherein the processor is further caused to perform:

presenting the heart rate value on a display located within the driving apparatus.

15. The system of any of claims 9 to 14, wherein the user is a driver of the driving apparatus; and wherein the processor is further caused to perform:

identifying a passenger of the driving apparatus;
receiving signals from a second bio-radar sensor, the second bio-radar sensor being equipped in a second seat of the driving apparatus and the signals include information regarding a heart rate of the passenger;
in response to receiving the signals from the second bio-radar sensor, processing the signals to determine a heart rate value of the passenger;
comparing the heart value of the passenger with a predetermined heart rate threshold for the passenger;
determining whether the detected heart rate of the passenger has breached the predetermined heart rate threshold for the passenger; and

in response to determining the detected heart rate of the passenger has breached the predetermined heart rate threshold for the passenger, generate a second status indicating that the passenger is suffering an abnormal heart rate condition.

112a

Other Entity 112x

Notification 114 Regarding The Driving Apparatus (e.g., ID, Location, Operation Physical Status)

112b

108

Info 106

104

Info 106

102

FIG. 1

PASSENGER SEAT

DRIVER SEAT

204

206b

202

Processor
208

Display
210

206a

102

FIG. 2

Processor <u>208</u>

User Identification Component <u>302</u>

Signal Processing Component <u>304</u>

Heart Rate Processing Component <u>306</u>

Respiration Rate Processing Component <u>308</u>

Status Generation Component <u>310</u>

Communication Component <u>312</u>

FIG. 3

FIG. 4

Identifying A User Of The Driving Apparatus 502

Receiving Signals From A Bio-radar Sensor 504

Processing The Signals To Determine A Heart Rate And/or A Respiration Rate Of The User 506

Determining Whether The Detected Heart Rate And/or The Respiration Rate Of The User Have Breached The Predetermined Thresholds For The User 508

Generate A Status Indicating That The User Is Suffering An Abnormal Heart Rate And/or Respiration Rate Condition 510

500

FIG. 5

FIG. 6

Europäisches
Patentamt

European
Patent Office

Office européen
des brevets

**EUROPEAN SEARCH REPORT**

Application Number

EP 18 18 8586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2017 0055352 A (ESP CO LTD [KR]) 19 May 2017 (2017-05-19) * abstract; figures 1, 6 * * paragraph [0022] - paragraph [0024] * * paragraph [0027] - paragraph [0030] * * paragraph [0032] * | 1-15 | INV. G01S13/536 G01S13/88 A61B5/0205 |
| A | US 2009/203972 A1 (HENEGHAN CONOR [IE] ET AL) 13 August 2009 (2009-08-13) * abstract; figure 8 * * paragraphs [0015], [0017], [0045] * | 7,14 | |
| A | US 8 725 311 B1 (BREED DAVID S [US]) 13 May 2014 (2014-05-13) * abstract * * claims 1, 4, 12 * | 8,15 | |
| A | EP 3 069 916 A1 (THUNDER POWER HONG KONG LTD [CN]) 21 September 2016 (2016-09-21) * abstract; figures 7, 8 * * paragraph [0030] - paragraph [0035] * | 1,8 | |

TECHNICAL FIELDS
SEARCHED     (IPC)

G01S
A61B

The present search report has been drawn up for all claims

| Place of search | Date of completion of the search | Examiner |
|---|---|---|
| Munich | 7 December 2018 | Knoll, Bernhard |

EPO FORM 1503 03.82 (P04C01)

## ANNEX TO THE EUROPEAN SEARCH REPORT
## ON EUROPEAN PATENT APPLICATION NO.

EP 18 18 8586

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2018

| Patent document cited in search report | | Publication date | Patent family member(s) | | Publication date |
|---|---|---|---|---|---|
| KR 20170055352 | A | 19-05-2017 | NONE | | |
| US 2009203972 | A1 | 13-08-2009 | AU | 2007256872 A1 | 13-12-2007 |
| | | | CA | 2654095 A1 | 13-12-2007 |
| | | | CN | 101489478 A | 22-07-2009 |
| | | | EP | 2020919 A2 | 11-02-2009 |
| | | | HK | 1135868 A1 | 10-05-2013 |
| | | | JP | 2009538720 A | 12-11-2009 |
| | | | US | 2009203972 A1 | 13-08-2009 |
| | | | US | 2014163343 A1 | 12-06-2014 |
| | | | WO | 2007143535 A2 | 13-12-2007 |
| US 8725311 | B1 | 13-05-2014 | NONE | | |
| EP 3069916 | A1 | 21-09-2016 | CN | 105922950 A | 07-09-2016 |
| | | | CN | 206086596 U | 12-04-2017 |
| | | | CN | 206621356 U | 10-11-2017 |
| | | | CN | 207015287 U | 16-02-2018 |
| | | | CN | 207015288 U | 16-02-2018 |
| | | | CN | 207015289 U | 16-02-2018 |
| | | | EP | 3069916 A1 | 21-09-2016 |
| | | | US | 2016272214 A1 | 22-09-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 19926816 A
- US 97762715 A
- US 06343516 A